# EUROPEAN PATENT SPECIFICATION

(11) **EP 3 134 047 B1**
(45) Date of publication and mention of the grant of the patent: **07.10.2020**
(21) Application number: 15783332.8
(22) Date of filing: 16.04.2015
(51) Int. Cl.: A61F 13/49, A61F 13/51, A61F 13/514

(54) **STRETCH BREATHABLE PROTECTIVE ABSORBENT ARTICLE USING TRI-LAMINATE**
ATMUNGSAKTIVER DEHNBARER SAUGFÄHIGER SCHUTZARTIKEL MIT TRILAMINAT
ARTICLE ABSORBANT PROTECTEUR RESPIRANT EXTENSIBLE UTILISANT UN TRI-STRATIFIÉ

(30) Priority: 21.04.2014 US 201414257450
(43) Date of publication of application: 01.03.2017
(73) Proprietor: Medline Industries, Inc., Mundelein, IL 60060 (US)
(72) Inventor: NELSON, Christopher, Plymouth, WI 53073 (US)
(74) Representative: Gaunt, Thomas Derrick
(86) International application number: PCT/US2015/026119
(87) International publication number: WO 2015/164173

(56) References cited:
- WO-A2-2009/117492
- US-A1- 2008 287 899
- US-A1- 2010 168 705
- US-A1- 2011 098 668
- US-B2- 6 719 744
- US-B2- 7 534 237
- US-B2- 8 206 365
- US-B2- 8 292 865

## Description

### FIELD OF THE INVENTION

The present invention relates a disposable absorbent article in an underwear or pull-up style. More particularly, the invention relates to protective underwear that makes use of breathable laminate material having three layers.

### BACKGROUND OF THE INVENTION

Millions of people of all ages suffer from incontinence of the bowel or bladder. Whether an infant, adult, or elderly person, the underlying cause of incontinence varies but the method of treatment typically involves use of absorbent article products. Adult incontinent briefs, disposable diapers and underpads can alleviate some of the emotional and physical discomfort of incontinence by absorbing and containing liquid and other discharges from the human body to prevent body and clothing soiling.

A disadvantage of known disposable undergarments is that they are often constructed from materials that are designed to capture urine and other exudates and prevent leakage, but are not breathable. Consequently, moisture may become trapped between the wearer and the disposable undergarment leading to discomfort and irritation. Further, as disposable undergarments are intended to replace traditional undergarments, disposable undergarments must be constructed to permit the wearer to be repeatedly put-on and pull-off the garment as necessary until such time as the garment is ready for disposal.

Disposable protective underwear products are known in the art. Such disposable underwear products rely on retractive forces that are provided by elastics, such as spandex strands. It is also known to use stretch elastic laminates that replace the spandex strands so as to provide better a fit to the wearer and improved discretion. Some products are created from a coextruded elastic layer made during the nonwoven manufacturing process to provide a product with improved breathability.

Widlund, et al., U.S. Patent No. 6,375,646 teaches a disposable diaper including an elongated absorbent pad, inner and outer casing layers and an elastically stretchable region in at least one of the front and back portions of the disposable diaper. The crotch portion of the disposable diaper is not stretchable. The combined stretchable and non-stretchable regions are designed to hold the absorbent material against the wearer's body to prevent leakage.

Norrby, et al., U.S. Patent No. 8,298,205 teaches an elastically stretchable laminate that includes a first non-elastic nonwoven web, a second non-elastic nonwoven web and an elastic film between the first and the second nonwoven webs. The laminate is rendered elastic in a first direction by incremental stretching and partial tearing of the first and second nonwoven webs.

Thorson, et al., U.S. Patent Application Publication No. 2011/0098668, teaches a disposable absorbent garment employing elastomeric film laminate body panels. The laminate can include an elastomeric film and nonwoven layers, and inner and outer surfaces adhered to nonwoven and elastomeric film layers.

Stablefeldt, et al., U.S. Patent Application Publication No. 2010/0168705, teaches disposable absorbent garments employing elastomeric film laminates with deactivated regions. A portion of the disposable garment includes laminated elastomeric and non-elastomeric polymeric film layers and a nonwoven layer. An absorbent member extends partially through the laminated layers.

Gilgenback U.S. Patent Application Publication No. 2010/0163161 teaches a process for making disposable absorbent garments employing elastomeric film laminates with deactivated regions. A portion of the disposable garment includes laminated elastomeric and non-elastomeric polymeric film layers and a nonwoven layer. An absorbent member extends partially through the laminated layers.

Kielpikowski, et al., U.S. Patent No. 4,842,596, teaches a method for making a breathable elastic fabric composite and personal article incorporating same. A liquid impermeable elastomeric film is sandwiched between pairs of nonwoven sheets. The elastomeric film is a partially stretched condition and bonded to the nonwoven sheets. The resulting laminated sheets create gathers that form breathable apertures.

Klemp, et al., U.S. Patent No. 6,994,761 teaches a disposable diaper and process for making the same. The diaper includes inner and outer portions that are ultrasonically bonded to create the vent sites or apertures through a layer of stretchable, breathable material.

Morrell-Schwartz, et al., U.S. Patent Application Publication No. 2008/0287899 teaches a disposable diaper including an ultrasonically bonded stretchable film.

According to an aspect of the present invention, there is provided an absorbent article, comprising: a front laminate section defining front end edge, a front crotch edge parallel to and longitudinally spaced from the front end edge, first and second transversely opposed side edges extending in a longitudinal directions, and first and second leg edges, the front laminate section comprising: a polymeric film, a first nonwoven layer attached to a first side of the polymeric film, a second nonwoven layer attached to a second side of the polymeric film opposite the first nonwoven layer, a front non-elastic portion adjacent to the front crotch edge, and a front elastic portion adjacent to the front end edge; a leg elastic that is adhered to a surface of the front laminate section and is parallel and adjacent to the first leg edge for at least a portion of the length of the leg elastic; a rear laminate section defining rear end edge, a rear crotch edge parallel to and longitudinally spaced from the rear end edge, and first and second transversely opposed side edges extending in a longitudinal directions, the rear laminate section comprising a rear non-elastic portion adjacent to the rear crotch edge and a rear elastic portion adjacent to the rear end edge; and an absorbent assembly extending longitudinally between the front crotch edge and the rear crotch edge, the absorbent assembly comprising a topsheet, a backsheet and an absorbent core positioned between the topsheet and backsheet; wherein the absorbent core overlaps with the front non-elastic portion and the rear non-elastic portion and does not overlap with the front elastic portion and the rear elastic portion; characterized in that the first and second nonwoven layers are attached to the polymeric film at a plurality of spaced apart ultrasonic bonding sites, wherein the bonding sites comprise a plurality of through passages that provide for the passage of water vapor through the front laminate ; and further comprising a covering layer that is adhered to a surface of the front laminate section and covers the leg elastic.

### BRIEF DESCRIPTION OF THE FIGURES

Advantages of the invention will become apparent upon reading the following detailed description and upon reference to the drawings.
FIG. 1 is a top plan view of laminate portions of an absorbent article in a substantially flat uncontracted position according to an embodiment of the invention.
FIG. 2 is a cross-sectional view of the laminate of FIG. 1 along sectional line A-A.
FIG. 3 is a cross-sectional view of a breathable laminate according to an embodiment of the invention.
FIG. 4 is a top view of the laminate of FIG. 3.
FIG. 5 is a top plan view of an embodiment of the absorbent article of FIG. 1 in a substantially flat un-contracted position and further including leg and waist elastics.
FIG. 6 is an alternative embodiment of the absorbent article of FIG. 5.
FIG. 7 is a top plan view of an embodiment of the absorbent article of FIG. 1 in a substantially flat un-contracted position and further including a covering nonwoven layer.
FIG. 8 is a cross-sectional view of the absorbent article of FIG. 7 along sectional line B-B.
FIG. 9 is a top plan view of an embodiment of the absorbent article of FIG. 1 in a substantially flat un-contracted position and further including an absorbent assembly.
FIG. 10 is an exploded perspective view of an embodiment of the absorbent article of FIG. 9.

### DETAILED DESCRIPTION

Absorbent articles as described herein generally include a moisture-pervious inner layer, an absorbent layer, and a moisture-impervious outer layer. Although the remainder of the description will be specifically directed to adult incontinence articles, such as disposable diapers or briefs, it is to be understood that the embodiments may also be implemented using other absorbent articles and that the properties and uses described below apply to these other absorbent articles as well. Throughout this application, the terms absorbent article, diaper or brief are used interchangeably. However, it should be understood that the terms diaper or brief are intended to include other absorbent articles, such as training pants, incontinence pads, etc., as would be understood by one of ordinary skill in the art.

As used in the description herein and throughout the claims, the following terms take the meanings explicitly associated herein, unless the context clearly dictates otherwise: the meaning of "a," "an," and "the" includes plural reference, the meaning of "in" includes "in" and "on." Relational terms such as first and second, top and bottom, proximal and distal, and the like may be used solely to distinguish one entity or action from another entity or action without necessarily requiring or implying any actual such relationship or order between such entities or actions.

As used herein, the following terms have the following meanings:
"Attach" and its derivatives refer to the joining, adhering, connecting, bonding, sewing together, or the like, of two elements. Two elements will be considered to be attached together when they are integral with one another or attached directly to one another or indirectly to one another, such as when each is directly attached to intermediate elements. "Attach" and its derivatives include permanent, releasable, or refastenable attachment. In addition, the attachment can be completed either during the manufacturing process or by the end user.

"Bond" and its derivatives refer to the joining, adhering, connecting, attaching, sewing together, or the like, of two elements. Two elements will be considered to be bonded together when they are bonded directly to one another or indirectly to one another, such as when each is directly bonded to intermediate elements. "Bond" and its derivatives include permanent, releasable, or refastenable bonding.

"Connect" and its derivatives refer to the joining, adhering, bonding, attaching, sewing together, or the like, of two elements. Two elements will be considered to be connected together when they are connected directly to one another or indirectly to one another, such as when each is directly connected to intermediate elements. "Connect" and its derivatives include permanent, releasable, or refastenable connection. In addition, the connecting can be completed either during the manufacturing process or by the end user.

"Breathable" when used in describing a layer or multi-layer laminate means that the layer has the ability to allow moisture vapor to be transmitted through the material. Breathable layers may be air permeable, but it is not necessary to be air permeable to be breathable. In addition, breathable layers may be liquid permeable or liquid impermeable.

"Disposable" refers to articles that are designed to be discarded after a limited use rather than being laundered or otherwise restored for reuse.

The terms "disposed on," "disposed along," "disposed with," or "disposed toward" and variations thereof are intended to mean that one element can be integral with another element, or that one element can be a separate structure bonded to or placed with or placed near another element.

"Fiber" refers to a continuous or discontinuous member having a high ratio of length to diameter or width. Thus, a fiber may be a filament, a thread, a strand, a yarn, or any other member or combination of these members.

"Layer" when used in the singular can have the dual meaning of a single element or a plurality of elements.

"Liquid impermeable," when used in describing a layer or multi-layer laminate means that liquid, such as urine, will not pass through the layer or laminate, under ordinary use conditions, in a direction generally perpendicular to the plane of the layer or laminate at the point of liquid contact.

"Liquid permeable" refers to any material that is not liquid impermeable.

"Member" when used in the singular can have the dual meaning of a single element or a plurality of elements.

"Nonwoven" and "nonwoven web" refer to materials and webs of material that are formed without the aid of a textile weaving or knitting process. For example, nonwoven materials, fabrics or webs have been formed from many processes such as meltblowing processes, spunbonding processes, air laying processes, and bonded carded web processes.

These terms may be defined with additional language elsewhere in the specification.

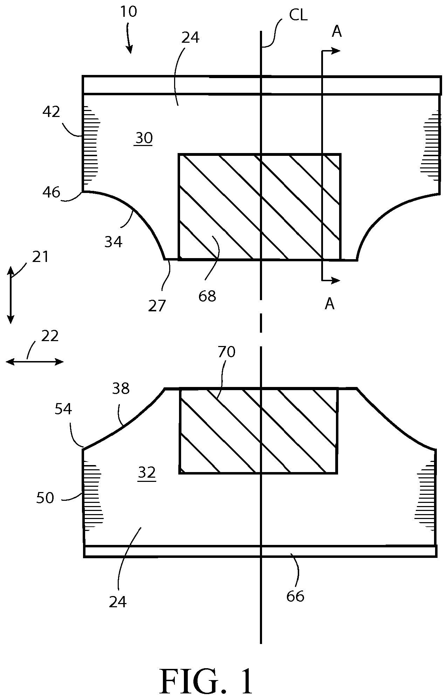

FIGS. 1, 5-7 and 9 illustrate a plan view of the absorbent article 10 in a substantially flat un-contracted state. As shown in these figures, the absorbent article 10 generally consists of several layers, including an inner layer, an absorbent layer, and an outer layer. The inner layer faces a wearer and contacts the skin of the wearer when the absorbent article 10 is secured to the wearer. The inner layer may comprise a topsheet that is composed of a moisture-pervious fabric suitable to allow bodily discharge to pass through the inner layer and be absorbed by the absorbent layer. Non-limiting examples of materials suitable to form the topsheet include polypropylene, polyethylene, polyester, materials having hydrophobic properties, combinations thereof and/or the like. Additionally, the topsheet can be treated with a hydrophilic finish to improve pass through of liquids to diaper layers beneath the inner layer. Non-limiting examples of suitable hydrophilic finishes include stearic acid, melamine-based chemicals, fluorocarbon chemicals, and silicon based chemicals.

The plan view of FIGS. 1, 5-7 and 9 is shown from the top or patient contacting side of the absorbent article. As illustrated in these figures, a particular embodiment of a disposable absorbent article 10 of the present invention defines a longitudinal direction 21 parallel to a centerline CL and a transverse direction 22 perpendicular to the longitudinal direction. The absorbent article comprises a front section 12, a rear section 16, and a crotch section 14.

Referring to FIG. 1, the absorbent article includes a film layer 24 comprising a laminate film. The laminate film may be divided into two sections such that the film layer 24 forms at least part of the front section 12 and rear section 16. The front film section 30 is spaced apart from the rear film section 32 such that they are separated in the crotch section 14.

The front film section 30 defines a front end edge 26 and a front crotch edge 27 parallel to and longitudinally spaced from the front end edge 26. The rear film section 32 defines a rear end edge 28 longitudinally opposite the front end edge 26 and a rear crotch edge 29 parallel to and longitudinally spaced from the rear end edge 28. The front film section defines opposed front leg edges 34 and 36, and the rear film section defines opposed rear leg edges 36 and 38.

The front film section 30 further defines first and second transversely opposed front side edges 42 and 44. The first front side edge 42 extends in the longitudinal direction 21 from the front end edge 26 to a front intersection point 46 where the first front side edge intersects the first front leg edge 34. The second front side edge 42 extends in the longitudinal direction 21 from the front end edge 26 to a front intersection point 48 where the first front side edge intersects the second front leg edge 36. The rear film section 32 also defines first and second transversely opposed back side edges 50 and 52. The first back side edge 50 extends in the longitudinal direction 21 from the back end edge 28 to a rear intersection point 54 where the first rear side edge 50 intersects the first rear leg edge 38, and the second back side edge 52 extends in the longitudinal direction 21 from the back end edge 28 to a rear intersection point 56 where the second rear side edge 52 intersects the second rear leg edge 40.

In particular embodiments, the front section 30 is constructed at least in part of a laminate 24 that comprises a polymeric film layer 62 and at least one nonwoven layer 60, wherein both the polymeric film layer 62 and the nonwoven layer 60 extend substantially throughout the area of the laminate 24.

In its completed form as used by a wearer, the absorbent article includes a first side seam at which the first front side edge 42 is attached to the first back side edge 50 and which defines a first side seam length. The article further includes a second side seam at which the second front side edge 44 is attached to the second back side edge 52 and which defines a second side seam length. The article is accordingly formed into a brief or pull-up style disposable absorbent article.

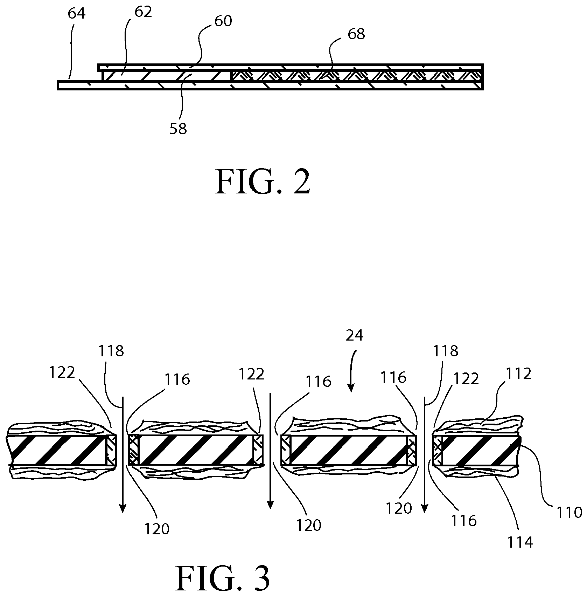

FIG. 2 shows a cross-sectional view of the laminate 24 along line A-A. In one embodiment, the laminate 24 comprises two nonwoven layers 58, 60 superposed on opposing bottom and top surfaces of the polymeric film 62 such that the polymeric film 62 is sandwiched between the two nonwoven facings 58, 60, and both the polymeric film 62 and both nonwoven layers 58, 60 extend substantially through the area of the laminate 24. The polymeric film layer may be a block copolymer. A portion 64 of the bottom or outer layer of nonwoven 58 may extend beyond the polymeric film 62 along front end edge 26. A further portion 66 of the bottom or outer layer of nonwoven 58 may extend beyond the polymeric film 62 along rear end edge 28.

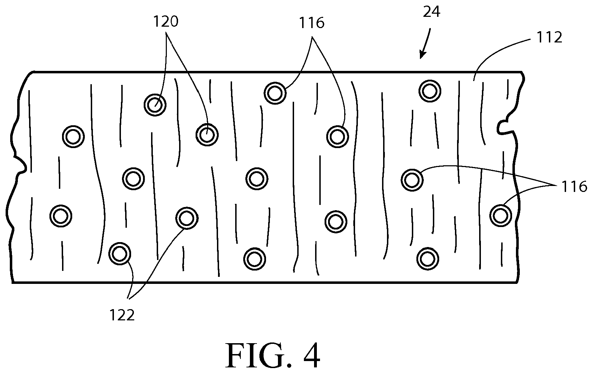

As shown in FIGS. 3-4, the laminate 24 may be formed of a breathable cloth-like elastic nonwoven laminar fabric by sandwiching a liquid impermeable and non-self-adhering elastomeric film or nonwoven carrier sheet 110 between at least a pair of nonwoven facing sheets 112, 114 and bonding the facing sheets 112, 114 together by autogenous bonds, such as ultrasonically or thermally-generated bonds, through the carrier sheet 110 at spaced apart sites 116, thereby forming breathable apertures 120 through the carrier sheet which laminate the carrier and facing sheets together at the spaced apart sites 116.

In an embodiment of the present invention, the facing sheets 112, 114 and the elastomeric film 110 are ultrasonically bonded at sites 116. The ultrasonic bonding process creates a bond region 122 where the material from the top sheet 112 and the bottom sheet 114 mix together to form a bond. The bond region may also include mixing of the elastomeric film 110 material such that all three layers are bonded together. The ultrasonic bonding process may be configured such that it generates a through passage 120 generally within the confines of the bond region 122 in order to provide for the passage of water vapor 118 and give breathability to the laminate 24.

Alternatively, the laminate 24 can also be constructed such that the web is not made breathable during the ultrasonic laminating process, but rather has breathability imparted through a needling, slitting or die treatment process after formation of the complete laminate.

Referring again to FIG. 1, at least a portion 68 of the front film section 30 is non-elastomeric, and at least a portion 70 of the rear film section 32 is non-elastomeric. In FIGS. 1, 5-7 and 9 non-elastomeric or partially elastomeric regions are indicated by a pattern of hash lines, which lines are continuous if the non-elastomeric regions are exposed, and which lines are dashed if the non-elastomeric regions are concealed by an overlying component. In one preferred approach, as shall be described in more detail below, the entire laminate 24 is constructed of an elastomeric film laminate which includes an elastomeric film layer and at least one nonwoven facing layer, and a portion of the laminate has been "deactivated" or "deadened" to render it non-elastomeric.

As used herein, "elastomeric" refers to a material or composite which can be elongated by at least 50 percent of its relaxed length and which will recover, upon release of the applied force, at least 50 percent of its elongation. It is generally preferred that the elastomeric material or composite be capable of being elongated by at least 100 percent, more preferably by at least 200 percent, of its relaxed length and recover, upon release of an applied force, at least 50 percent of its elongation. "Non-elastomeric" refers to a material or composite that is non-extensible, or that is extensible but will recover no more than 20 percent of its elongated length after release of an applied elongating force. "Non-extensible" refers to a material that cannot stretch or extend by more than 25 percent of its relaxed length without fracture upon application of a biasing force. "Partially elastomeric" refers to a material or composite which can be elongated by at least 50 percent of its relaxed length and which will recover, upon release of the applied force, more than 20 percent but less than 50 percent of its elongation.

"Deactivated" as used herein to describe a material, region of a material, or regions of a material means that the material, region, or regions of material has been treated in some way to substantially destroy the elastic properties of the material, region, or regions, rendering the material, region, or regions non-elastomeric.

Deactivation of the non-elastic portions 68, 70 may be accomplished by a deactivation unit to create deactivated regions in the elastomeric film laminate 24. The deactivation can be accomplished by any of a variety of means. Frequently, some form of energy is applied to deactivate the non-elastic regions 68, 70, such as pressure, heat, ultrasonic energy, combinations thereof, and the like. Techniques employing pressure, heat, and ultrasonic energy are known in the art. The deactivation can occur in a variety of patterns. For example, the deactivating energy could be applied in a solid pattern, a series of vertical stripes, horizontal stripes, or diagonal stripes, a series of squares or dots, or other suitable pattern.

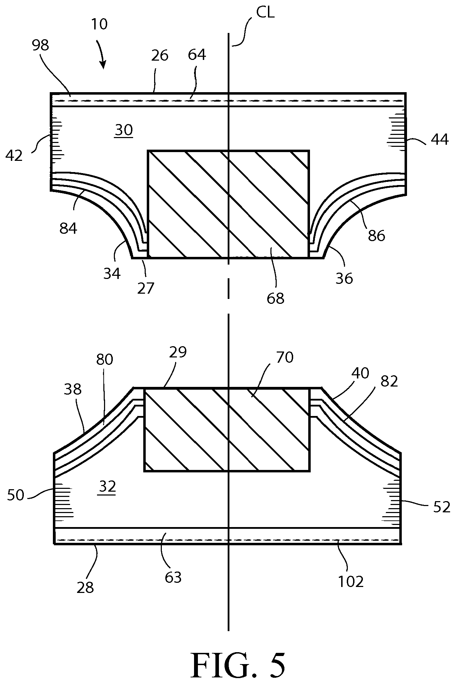

In embodiments of the present invention as illustrated in FIG. 5, the absorbent article 10 comprises a first rear leg elastic member 80 attached to an inside surface of the rear film section 32 adjacent at least a portion of the first rear leg edge 38, and a second back leg elastic member 82 to an inside surface of the rear film section 32 adjacent at least a portion of the second rear leg edge 40. In further embodiments, the absorbent article 10 comprises a first front leg elastic member 84 attached to attached to an inside surface of the rear film section 30 adjacent at least a portion of the first front leg edge 34, and a second front leg elastic member 86 attached to attached to an inside surface of the rear film section 30 adjacent at least a portion of the second front leg edge 36. Each leg elastic member 80, 82, 84, 86 can comprise a single strand, ribbon, or strip of elastomeric material, or each can comprise two or more strands, ribbons, or strips, such as, for example, three strands (as depicted in FIG. 5). The leg elastic members 80, 82, 84, 86 may be glued in place or otherwise adhered to a top surface of nonwoven layer 60.

As illustrated in FIG. 5, rear leg elastic member 80, 82 may extend from side edges 50, 52 of the rear film section 32 along rear leg edges 38, 40 to side edges of the non-elastic portion 70 of the rear film section. Alternatively, the rear leg elastic member 80, 82 may extend across part or the entire non-elastic portion 70. Likewise, front leg elastic member 84, 86 may extend from side edges 42, 44 of the front film section 30 along front leg edges 34, 36 to side edges of the non-elastic portion 68 of the front film section. Alternatively, the front leg elastic member 84, 86 may extend across part or the entire non-elastic portion 68.

For example, the first rear leg elastic member 80 and the second rear leg elastic member 82 may form part of a single, integral back elastic member that extends from the first rear side edge 50 transversely over the non-elastic portion 70 to the second rear side edge 52. Similarly, in certain embodiments, the first front leg elastic member 84 and the second front leg elastic member 86 form part of a single, integral front elastic member that extends from the first front side edge 42 transversely over the non-elastic portion 68 to the second front side edge 44.

In embodiments, as illustrated in FIG. 5, the extension 64 of the outer nonwoven layer 58 (see FIGS. 1-2) of the front portion 30 may be folded over the top of inner nonwoven layer 60 to define the front end edge 26. A similar extension 63 of the outer layer of nonwoven of the rear laminate 32 may be folded over the top of inner nonwoven layer to define the rear end edge 28.

The absorbent article 10 may further include a front waist elastic member 98 positioned within the front fold 64 and a back waist elastic member 102 positioned within the back fold 63. In alternative embodiments, no front waist fold or back waist fold is included; in such embodiments, opposite end edges of the laminate sections 30, 32 would define the front end edge 26 and back end edge 28, respectively. Each waist elastic member 98, 102 may comprise a single strand, ribbon, or strip of elastomeric material, or each can comprise two or more strands, ribbons, or strips.

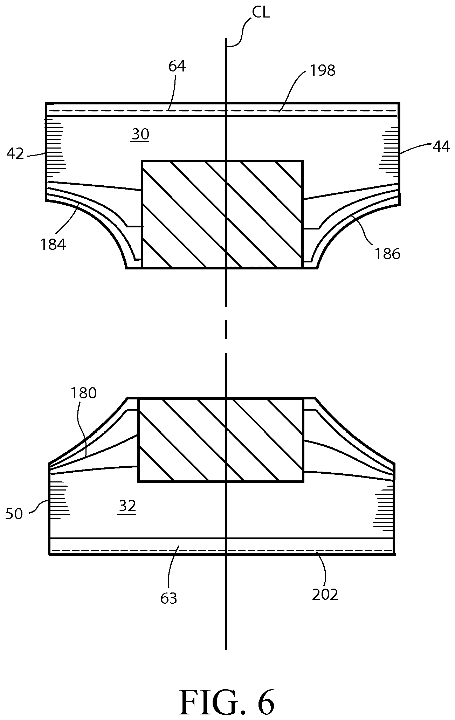

FIG. 6 illustrates further embodiments in which the leg elastic members 180, 182, 184, 186 comprises a series of elastic strands. The illustrated embodiment shows three such strands, but more or fewer strands may be used. The leg elastics may be applied in a curved fashion. At the side edges 42, 44, 50, 52 of the diaper, the leg elastics are generally parallel, and each of the independent leg elastics are then curved towards the respective non-elastic portions 68, 70 of the film sections 30, 32, and increasingly separated in distance from one another the closer the leg elastics get to the non-elastic film portions. Also as shown in FIG. 6, the waist elastic members 198, 202 may comprise multiple elastic strands.

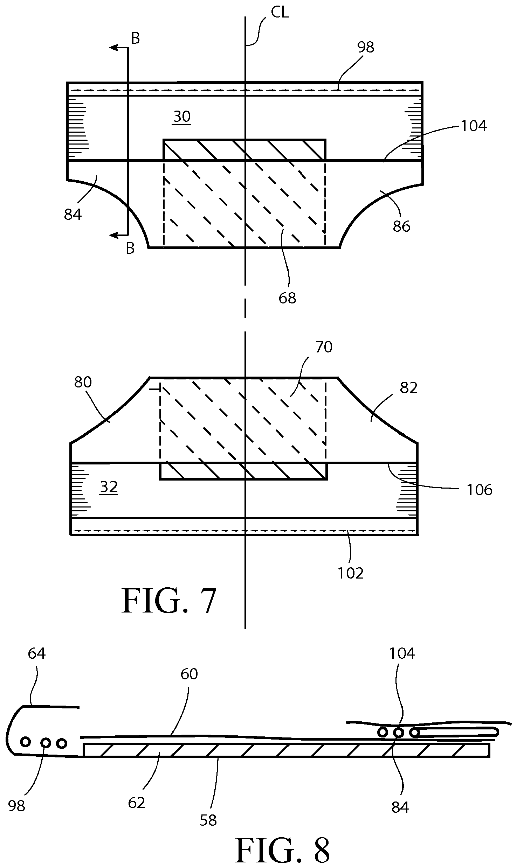

As illustrated in FIGS. 7-8, an additional covering nonwoven layer 104 may be attached to a top surface of the nonwoven layer 60 that comprises the top layer of the front laminate section 30. Additionally, a further covering nonwoven layer 106 may be attached to a top surface of the nonwoven layer 60 that comprises the top layer of the rear laminate section 32. The covering nonwoven layers 104, 106 are placed on top of laminate sections so that they at least in part cover the leg elastic elements. The covering nonwoven layers may be adhesively bonded to the film laminate 24 in the region where the leg elastics 80, 82, 84, 86 are not present and may be glued to the elastics and/or the top laminate nonwoven layer 60 in regions where the elastics are located.

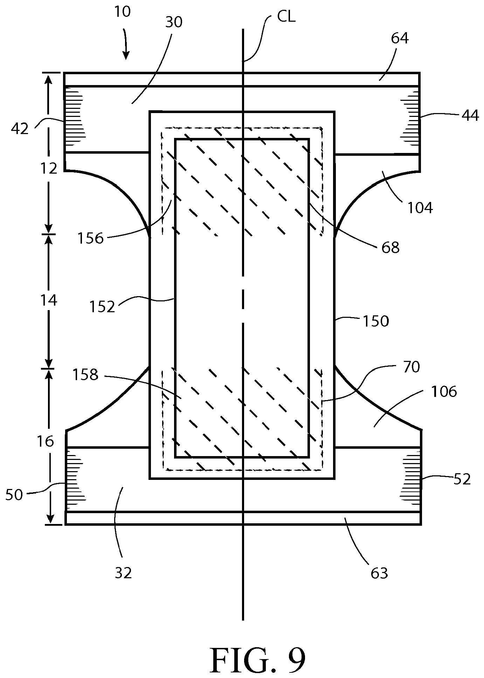

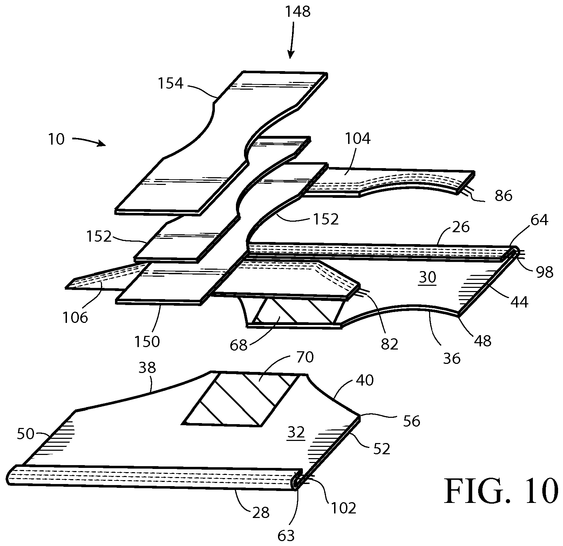

As illustrated in FIGS. 9-10, the absorbent article 10 also includes an absorbent assembly 148 that extends from the front section 12, across the crotch section 14, to the rear section 16. The absorbent assembly includes an absorbent core 152, and may include a topsheet 154 and a backsheet 150. (The topsheet 154 has been removed in FIG. 9 to more clearly show the position of the backsheet 150 and absorbent core 152.) The absorbent assembly 148 may be generally rectangular as shown in FIG. 9 or may comprise curved sections 166 to accommodate the wearer's legs as shown in FIG. 10. The absorbent core 152 may have an area that is smaller than the topsheet 154 and backsheet 150 such that the absorbent core is contained within the periphery of the absorbent assembly. The topsheet 154 and backsheet 150 may be bonded or otherwise adhered around a periphery of the absorbent assembly in order to capture the absorbent core 152 between the two sheets.

As shown in FIG. 9, the absorbent assembly 148 overlaps with the front section 30 to form a front overlapping zone 156, and the absorbent assembly 148 overlaps with the rear section 32 to form a rear overlapping zone 158. The periphery of the absorbent core 152 may be positioned completely within the front non-elastic portion 68 where the core overlaps with the front film section 30 and positioned completely within the rear non-elastic portion 70 where the core overlaps with the front film section 32. The backsheet 150 and topsheet (not shown) may also be positioned within the non-elastic portions 68, 70 in the overlapping zones, or may extend beyond the non-elastic portions as illustrated in FIG. 9.

In embodiments of the invention, the non-elastic portions 68, 70 may encompass more than 50% of the respective overlapping zones 156, 158. In other embodiments, more than 75%, and in further embodiments more than 90% of the area of the overlapping zones 156, 158 are non-elastomeric. In further embodiments, the entire of the overlapping zones are non-elastomeric. By adjusting the size of the non-elastic portions 68, 70 relative to the size of the absorbent core 152, the fit range of the article may be adjusted or the shape of the absorbent assembly may be defined in order to more readily capture exudate or prevent leaks.

In further embodiments, the non-elastic portions 68, 70 extend beyond the periphery of the absorbent core 152 or even beyond the backsheet 150. For example, non-elastic regions may be at least 10% larger, 20% larger, or in further embodiments 25% larger in area than the respective overlapped regions 156, 158. Providing non-elastic portions that are larger than their respective overlapping zones allows the process to accommodate any registration variability that may be present in the manufacturing process.

While the present invention has been described with reference to one or more particular embodiments, those skilled in the art will recognize that many changes may be made thereto without departing from the scope of the claims. Furthermore, components from one embodiment can be used in other non-exclusive embodiments within the scope of the claims.

## Claims

1. An absorbent article (10), comprising:
a front laminate section (30) defining front end edge (26), a front crotch edge (27) parallel to and longitudinally spaced from the front end edge (26), first and second transversely opposed side edges (42,44) extending in a longitudinal directions, and first and second leg edges (34,36),
the front laminate section (30) comprising:
a polymeric film (62),
a first nonwoven layer (60) attached to a first side of the polymeric film (62),
a second nonwoven layer (58) attached to a second side of the polymeric film (62) opposite the first nonwoven layer (60),
a front non-elastic portion (68) adjacent to the front crotch edge (27), and
a front elastic portion (98) adjacent to the front end edge (26);
a leg elastic (84) that is adhered to a surface of the front laminate section (30) and is parallel and adjacent to the first leg edge (34) for at least a portion of the length of the leg elastic (84);
a rear laminate section (32) defining rear end edge (28), a rear crotch edge (29) parallel to and longitudinally spaced from the rear end edge (28), and first and second transversely opposed side edges (50,52) extending in a longitudinal directions, the rear laminate section (32) comprising a rear non-elastic portion adjacent (70) to the rear crotch edge (29) and a rear elastic portion (102) adjacent to the rear end edge (28); and
an absorbent assembly (148) extending longitudinally between the front crotch edge (27) and the rear crotch edge (29), the absorbent assembly comprising a topsheet (154), a backsheet (150) and an absorbent core (152) positioned between the topsheet (154) and backsheet (150);
wherein the absorbent core (152) overlaps with the front non-elastic portion (68) and the rear non-elastic portion (70) and does not overlap with the front elastic portion (98) and the rear elastic portion (102);
**characterized in that** the first and second nonwoven layers (60,58) are attached to the polymeric film (62) at a plurality of spaced apart ultrasonic bonding sites (116), wherein the bonding sites (116) comprise a plurality of through passages (120) that provide for the passage of water vapor through the front laminate (30); and further comprising a covering layer (104) that is adhered to a surface of the front laminate section (30) and covers the leg elastic (84).

2. The absorbent article of claim 1, wherein the polymeric film (62) is an elastomeric film.

3. The absorbent article of claim 1, wherein the polymeric film (62) is a block copolymer.

4. The absorbent article of claim 2, wherein the non-elastic portion (68) of the front laminate (30) is formed by deactivating a portion of the elastomeric film.

5. The absorbent article of claim 1, wherein each through passages (120) is within the perimeter of a bonding site (116).

6. The absorbent article of claim 1, wherein the ultrasonic bonding sites (116) comprise a mixture of material from the first nonwoven layer (60), the polymeric film (62) and the second nonwoven layer (58) such that the first nonwoven layer (60), the polymeric film (62) and the second nonwoven layer (58) are bonded together.

7. The absorbent article of claim 1, wherein the front laminate (30) comprises a plurality of through passages (116) that provide for the passage of water vapor through the front laminate (30).

8. The absorbent article of claim 7, wherein the plurality of through passages (116) comprise perforations formed by passing a perforating tool through the front laminate(30).

9. The absorbent article of claim 1, wherein the rear laminate section (32) further comprises layers corresponding to layers of the front laminate section (30), including:
a polymeric film (62),
a first nonwoven layer (60) attached to a first side of the polymeric film (62), and
a second nonwoven layer (58) attached to a second side of the polymeric film (62) opposite the first nonwoven layer (60);
the rear laminate section (32) further defines first and second leg edges (34,36);
and the absorbent article (10) further comprises:
a rear leg elastic (80) adhered to a surface of the rear laminate section (32) and extending parallel and adjacent to the first rear leg edge (38) for at least a portion of the length of the rear leg elastic (80);
a rear covering layer (106) that is adhered to a surface of the rear laminate section (32) and covers the rear leg elastic (80),
wherein the rear laminate first and second nonwoven layers (60,58) are attached to the polymeric film (62) at a plurality of spaced apart ultrasonic bonding sites (116).

## Patentansprüche

1. Ein absorbierender Artikel (10), umfassend:
Einen vorderen Schichtstoffabschnitt (30), der einen vorderen Rand (26), einen vorderen Schritt-Rand (27), parallel und längs, mit Zwischenraum versehen, zu dem vorderen Rand (26), einen ersten und zweiten quer gegenüberliegenden Rand (42, 44), die sich in Längsrichtung erstrecken, und einen ersten und zweiten Bein-Rand (34, 36) definiert,
der vordere Schichtstoffabschnitt (30) umfasst: eine Polymerfolie (62),
eine erste Vliesschicht (60), die an einer ersten Seite der Polymerfolie (62) angebracht ist, eine zweite Vliesschicht (58), die an einer zweiten Seite der Polymerfolie (62) gegenüber der ersten Vliesschicht (60) angebracht ist,
einen vorderen nichtelastischen Abschnitt (68) am vorderen Schritt-Rand (27) und einen vorderen elastischen Abschnitt (98) am vorderen Rand (26);
einen Bein-Gummizug (84), der an einer Oberfläche des vorderen Schichtstoffabschnitts (30) befestigt ist und parallel und anliegend an den ersten Bein-Rand (34) über mindestens einen Abschnitt der Länge des Bein-Gummizugs (84) verläuft;
einen hinteren Schichtstoffabschnitt (32), der einen hinteren Rand (28), einen hinteren Schritt-Rand (29), parallel und längs, mit Zwischenraum versehen, zum hinteren Rand (28) und einen ersten und zweiten quer gegenüberliegenden Rand (50, 52), die sich in Längsrichtung erstrecken, definiert, der hintere Schichtstoffabschnitt (32) umfasst einen hinteren nichtelastischen Abschnitt, anliegend an (70) den hinteren Schritt-Rand (29), und einen hinteren elastischen Abschnitt (102), anliegend an den hinteren Rand (28); und
eine absorbierende Anordnung (148), die sich längs zwischen dem vorderen Schritt-Rand (27) und dem hinteren Schritt-Rand (29) erstreckt, die absorbierende Anordnung umfasst eine Deckschicht (154), eine Unterschicht (150) und eine absorbierende Kernschicht (152), die zwischen der Deckschicht (154) und Unterschicht (150) positioniert ist;
wobei die absorbierende Kernschicht (152) den vorderen nichtelastischen Abschnitt (68) und den hinteren nichtelastischen Abschnitt (70) überlappt, aber den vorderen elastischen Abschnitt (98) und den hinteren elastischen Abschnitt (102) nicht überlappt;
**gekennzeichnet dadurch, dass** die erste und zweite Vliesschicht (60,58) an der Polymerfolie (62) an einer Vielzahl mit Zwischenräumen versehenen Ultraschall-Bindungsstellen (116) befestigt sind,
wobei die Bindungsstellen (116) eine Vielzahl von Durchgangsöffnungen (120) umfassen, die den Wasserdampf durch den vorderen Schichtstoff (30) leiten; und ferner eine Außenschicht (104) umfassen, die an einer Oberfläche des vorderen Schichtstoffabschnitts (30) befestigt ist und den Bein-Gummizug (84) abdeckt.

2. Der absorbierende Artikel nach Anspruch 1, wobei die Polymerfolie (62) eine Elastomerfolie ist.

3. Der absorbierende Artikel nach Anspruch 1, wobei die Polymerfolie (62) ein Blockpolymer ist.

4. Der absorbierende Artikel nach Anspruch 2, wobei der nichtelastische Abschnitt (68) des vorderen Schichtstoffs (30) durch die Deaktivierung eines Abschnitts der Elastomerfolie gebildet wird.

5. Der absorbierende Artikel nach Anspruch 1, wobei jede Durchgangsöffnung (120) innerhalb des Umfangs einer Bindungsstelle (116) liegt.

6. Der absorbierende Artikel nach Anspruch 1, wobei die Ultraschall-Bindungsstellen (116) eine Mischung aus Material aus der ersten Vliesschicht (60), der Polymerfolie (62) und der zweiten Vliesschicht (58) umfasst, so dass die erste Vliesschicht (60), die Polymerfolie (62) und die zweite Vliesschicht (58) miteinander verbunden sind.

7. Der absorbierende Artikel nach Anspruch 1, wobei der vordere Schichtstoff (30) eine Vielzahl von Durchgangsöffnungen (116) umfasst, die Wasserdampf durch den vorderen Schichtstoff (30) leiten.

8. Der absorbierende Artikel nach Anspruch 7, wobei die Vielzahl der Durchgangsöffnungen (116) Perforierungen umfasst, die dadurch geformt wurden, dass ein Perforationswerkzeug durch den vorderen Schichtstoff (30) geführt wurde.

9. Der absorbierende Artikel nach Anspruch 1, wobei der hintere Schichtstoffabschnitt (32) ferner Schichten umfasst, die den Schichten des vorderen Schichtstoffabschnitts (30) entsprechen, einschließlich:
einer Polymerfolie (62),
einer ersten Vliesschicht (60), die an einer ersten Seite der Polymerfolie (62) angebracht ist, und einer zweiten Vliesschicht (58), die an einer zweiten Seite der Polymerfolie (62) gegenüber der ersten Vliesschicht (60) angebracht ist;
der hintere Schichtstoffabschnitt (32) definiert ferner einen ersten und zweiten Bein-Rand (34, 36); und der absorbierende Artikel (10) umfasst ferner:
einen hinteren Bein-Gummizug (80), der an einer Oberfläche des hinteren Schichtstoffabschnitts (32) befestigt ist und parallel und anliegend an den ersten hinteren Bein-Rand (38) über mindestens einen Abschnitt der Länge des hinteren Bein-Gummizugs (80) verläuft;
eine hintere Außenschicht (106), die an einer Oberfläche des hinteren Schichtstoffabschnitts (32) angebracht ist und den hinteren Bein-Gummizug (80) abdeckt,
wobei die erste und zweite Vliesschicht (60,58) des hinteren Schichtstoffs an der Polymerfolie (62) an einer Vielzahl mit Zwischenräumen versehenen Ultraschall-Bindungsstellen (116) befestigt sind.

## Revendications

1. Article non absorbant (10), comprenant :
une section de stratifié avant (30) définissant le bord d'extrémité avant (26), un bord d'entrejambe avant (27) parallèle et espacé longitudinalement du bord d'extrémité avant (26), les premier et second bords latéraux en regard de manière transversale (42,44) s'étendant dans une direction longitudinale et les premier et second bords de jambe (34,36),
la section de stratifié avant (30) comprenant :
un film polymère (62),
une première couche non tissée (60) fixée à un premier côté du film polymère (62), une seconde couche non tissée (58) fixée à un second côté du film polymère (62) en regard de la première couche non tissée (60),
une partie avant non élastique (68) adjacente au bord d'entrejambe avant (27) et une partie avant élastique (98) adjacente au bord d'extrémité avant (26) ;
un élastique de jambe (84) qui est collé à une surface de la section de stratifié avant (30) et qui est parallèle et adjacent au premier bord de jambe (34) pour au moins une partie de la longueur de l'élastique de jambe (84);
une section de stratifié arrière (32) définissant le bord d'extrémité arrière (28), un bord d'entrejambe arrière (29) parallèle et espacé longitudinalement du bord d'extrémité arrière (28) et les premier et second bords latéraux en regard de manière transversale (50,52) s'étendant dans une direction longitudinale, la section de stratifié arrière (32) comprenant une partie arrière non élastique adjacente (70) au bord d'entrejambe arrière (29) et une partie arrière élastique (102) adjacente au bord d'extrémité arrière (28) ; et
un ensemble absorbant (148) s'étendant longitudinalement entre le bord d'entrejambe avant (27) et le bord d'entrejambe arrière (29), l'ensemble absorbant comprenant une feuille supérieure (154), une feuille arrière (150) et un cœur absorbant (152) placé entre la feuille supérieure (154) et la feuille arrière (150) ;
dans lequel le cœur absorbant (152) chevauche la partie avant non élastique (68) et la partie arrière non élastique (70) et ne chevauche pas la partie avant élastique (98) et la partie arrière élastique (102) ;
**caractérisé en ce que** les première et seconde couches non tissées (60,58) sont fixées au film polymère (62) à une pluralité de sites de collage par ultrasons espacés (116),
dans lequel les sites de collage (116) comprennent une pluralité de passages traversants (120) qui assurent le passage de la vapeur d'eau à travers le stratifié avant (30) ; et comprenant en outre une couche de couverture (104) qui est collée sur une surface de la section de stratifié avant (30) et couvre l'élastique de jambe (84).

2. Article absorbant selon la revendication 1, dans lequel le film polymère (62) est un film élastomère.

3. Article absorbant selon la revendication 1, dans lequel le film polymère (62) est un copolymère à blocs.

4. Article absorbant selon la revendication 2, dans lequel la partie non élastique (68) du stratifié avant (30) est constituée par la désactivation d'une partie du film élastomère.

5. Article absorbant selon la revendication 1, dans lequel chacun des passages traversants (120) se trouve à l'intérieur du périmètre d'un site de collage (116).

6. Article absorbant selon la revendication 1, dans lequel les sites de collage par ultrasons (116) comprennent un mélange de matériau provenant de la première couche non tissée (60), du film polymère (62) et de la seconde couche non tissée (58) de sorte que la première couche non tissée (60), le film polymère (62) et la seconde couche non tissée (58) sont collés ensemble.

7. Article absorbant selon la revendication 1, dans lequel le stratifié avant (30) comprend une pluralité de passages traversants (116) qui assurent le passage de la vapeur d'eau à travers le stratifié avant (30).

8. Article absorbant selon la revendication 7, dans lequel la pluralité de passages traversants (116) comprennent des perforations formées par le passage d'un outil de perforation à travers le stratifié avant (30).

9. Article absorbant selon la revendication 1, dans lequel la section de stratifié arrière (32) comprend en outre des couches correspondant aux couches de la section de stratifié avant (30), notamment :
un film polymère (62),
une première couche non tissée (60) fixée à un premier côté du film polymère (62) et une seconde couche non tissée (58) fixée à un second côté du film polymère (62) en regard de la première couche non tissée (60) ;
la section de stratifié arrière (32) définit en outre les premier et second bords de jambes (34,36) ;
et l'article absorbant (10) comprend en outre :
un élastique de jambe arrière (80) collé à une surface de la section de stratifié arrière (32) et s'étendant parallèle et adjacent au premier bord de jambe arrière (38) pour au moins une partie de la longueur de l'élastique de jambe arrière (80) ;
une couche de couverture arrière (106) qui est collée sur une surface de la section de stratifié arrière (32) et couvrant l'élastique de jambe arrière (80),
dans lequel les première et seconde couches non tissées de stratifié arrière (60,58) sont fixées au film polymère (62) à une pluralité de sites de collage par ultrasons espacés (116).
